# FASCICULE DE BREVET EUROPEEN

(11) **EP 1 495 776 B1**
(45) Date de publication et mention de la délivrance du brevet: **03.12.2008**
(21) Numéro de dépôt: 04291754.2
(22) Date de dépôt: 09.07.2004
(51) Int. Cl.: A61M 5/145

(54) **Dispositif pour équiper une seringue pour soumettre son piston à une pression positive automatique**
Vorrichtung um den Spritzenkolben einer Spritze automatisch unter positiven Druck zu setzen
Device for medical syringe for automatically subjecting its piston to a positive pressure

(30) Priorité: 11.07.2003 ES 200301669 U
(43) Date de publication de la demande: 12.01.2005
(73) Titulaire: VYGON, F-95440 Ecouen (FR)
(72) Inventeur: Suarez Delgado, José Manuel, 41500 Alcala de Guadaira (Sevilla) (ES); Carrez, Jean-Luc, 95440 Ecouen (FR); Dalle, Valéry, 60270 Gouvieux (FR); Brouillon, André, 60140 Rosoy (FR); Lesimple, Laurent, 75015 Paris (FR)
(74) Mandataire: Texier, Christian

(56) Documents cités:
- US-A- 4 381 006
- US-A- 5 024 662
- US-A- 5 531 696
- US-A- 5 643 213
- US-A- 5 944 693

## Description

L'invention concerne un dispositif pour équiper une seringue à usage médical, dont le corps présente à proximité de l'extrémité d'entrée du piston une extension latérale de préhension, par exemple des ailettes ou une collerette continue ou discontinue, et dont le piston présente une tête de préhension.

L'invention s'applique notamment aux seringues utilisées pour une injection dans l'espace péridural, en particulier une injection anesthésiante.

Elle s'applique à l'injection d'un volume gazeux (dit « mandrin gazeux ») ou d'un volume liquide (dit « mandrin liquide »).

L'opérateur qui veut réaliser une telle injection, détecte la pénétration de l'aiguille dans cet espace par le fait que la résistance à l'injection qui se manifestait auparavant cesse brusquement.
De ce fait, la pression qu'il exerçait sur le piston de la seringue pendant la recherche dudit espace devient efficace pour l'injection.

Cette recherche et le contrôle de cette pression mobilisent l'attention de l'opérateur au détriment de ses autres préoccupations.

On a proposé de soumettre le piston de la seringue à une pression positive continue constituée par une force de rappel élastique en sorte qu'une fois tiré pour le remplissage de la seringue et relâché, il revienne de lui-même à sa position initiale en l'absence de contre-pression.

Le modèle d'utilité espagnol U 9 802 777 décrit ainsi une solution dans laquelle la seringue est munie d'un lien élastique qui relie le corps de la seringue au piston en passant dans des encoches formées dans la tête du piston.

Ce dispositif très simple nécessite une certaine dextérité pour sa mise en place et nécessite une modification de la tête du piston, laquelle est normalement dépourvue de telles encoches dans le cas des seringues courantes du commerce.

Le document US 5 944 693 décrit un dispositif pour équiper une seringue permettant d'ajuster à la demande la longueur des tiges fixées à des ressorts.

Le document US 5 643 213 décrit un dispositif pour équiper une seringue comportant une bande élastique dont l'une des extrémités présente plusieurs trous échelonnés, permettant d'ajuster la longueur de la bande en enfilant l'un des trous sur une pièce réunissant les deux extrémités de ladite bande.

La présente invention a pour but de fournir un dispositif qui puisse équiper une seringue courante du commerce, qui soit d'une manipulation simple et qui soit fiable.

On y parvient, selon l'invention, avec un dispositif comme définit dans les revendications.

On décrira ci-après un exemple non limitatif de réalisation d'un tel dispositif, en référence aux figures du dessin joint sur lequel :
- la figure 1 est une perspective du dispositif ;
- la figure 2 est une perspective du dispositif monté sur une seringue à mandrin gazeux, le piston étant au repos ;
- la figure 3 est une perspective du dispositif monté sur une seringue à mandrin gazeux, le piston étant tiré, et
- la figure 4 est une perspective du dispositif monté sur une seringue à mandrin liquide, le piston étant au repos.

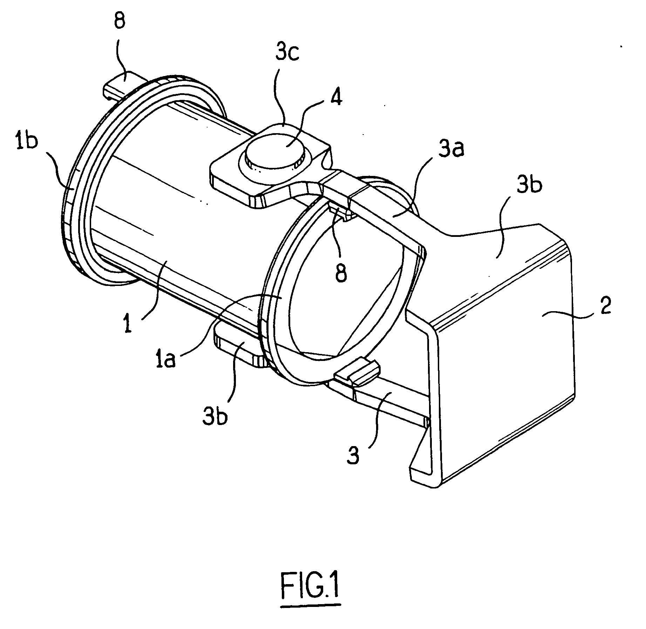

Le dispositif représenté en perspective sur la figure 1 est un corps unitaire qui comprend une embase cylindrique (1), un chapeau poussoir (2) et deux bras de liaison (3) montés en parallèle qui relient le chapeau au poussoir. De préférence, le chapeau et les deux bras viennent ensemble de fabrication.

A leurs extrémités libres, les bras sont fixés à l'embase. Dans l'exemple représenté, les bras présentent une partie centrale amincie (3a) entre deux extrémités (3b, 3c) pour la liaison des bras au chapeau et à l'embase.

Le chapeau (2) est prévu suffisamment large pour couvrir la tête du piston (7) de la seringue. De préférence, la face du chapeau qui est au contact de la tête du piston présente une collerette circulaire (2a) qui permet de centrer la tête du piston dans le chapeau.

L'embase est un fourreau a toute longueur voulue, et d'un diamètre qui convient pour coulisser sur le corps tubulaire (5) de la seringue jusqu'à venir en butée sur le col (6) de ce corps.

Dans l'invention, l'embase constitue un fourreau qui peut être enfilé sur le corps de la seringue, au choix par l'une ou l'autre des deux extrémités opposées (1a, 1b) du fourreau, et les emplacements (4) de fixation des bras (3) sur le fourreau sont plus proches d'une extrémité de l'embase que de l'autre et constituent des pivots sur lesquels peuvent tourner les bras.

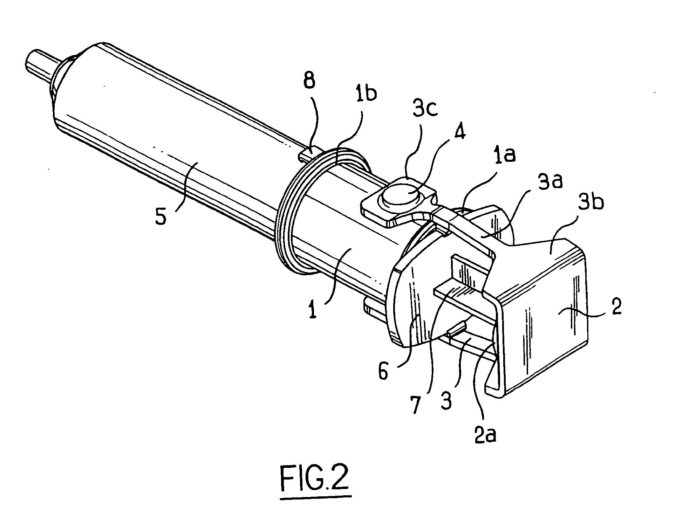

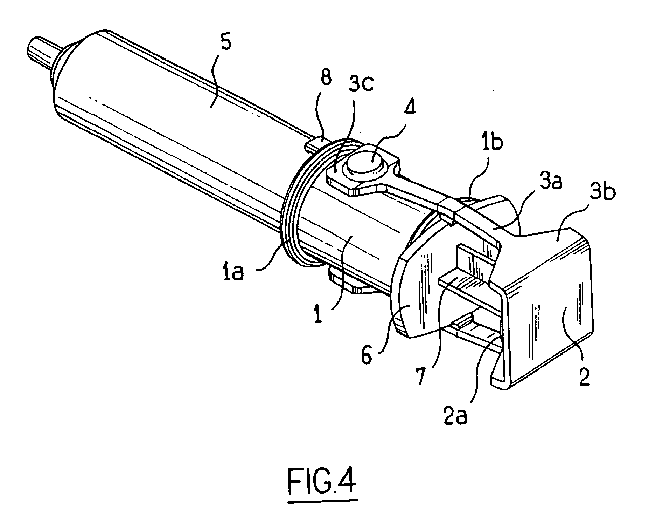

Cette disposition avantageuse permet, avec les mêmes bras, de choisir la distance des pivots au chapeau entre une distance courte et une distance longue, selon que l'embase a été enfilée sur le corps tubulaire par son extrémité (1a) proche des pivots ou par son extrémité (1b) éloignée des pivots. Dans le cas de la figure 2, la distance des pivots au chapeau est courte et, pour passer à la distance longue (figure 4), il suffit de retourner l'embase et de basculer les bras de 180°.

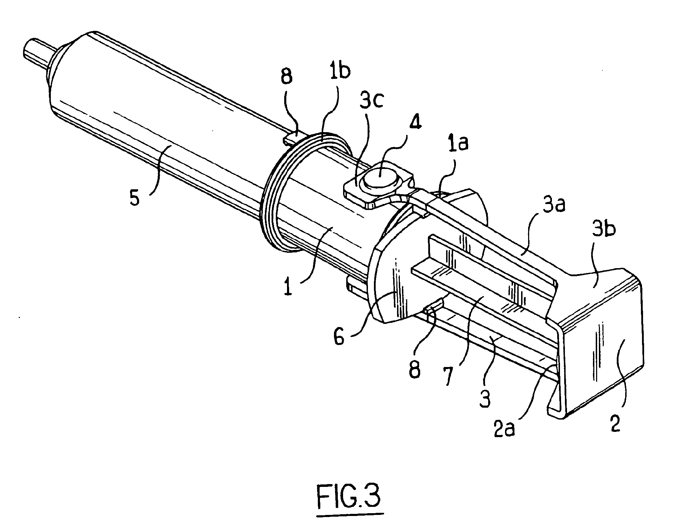

Le choix de la distance courte convient plus particulièrement au cas où le fluide à injecter est un gaz puisque la tension des bras élastiques n'a pas besoin d'être élevée pour l'expulsion du gaz et l'on a représenté sur les figures 2 et 3 une telle seringue équipée du dispositif de l'invention respectivement avec le piston (7) au repos (figure 2) et le piston tiré (figure 3).

Le choix de la distance longue convient plus particulièrement au cas où le fluide à injecter est un liquide qui nécessite une tension plus élevée des bras élastiques et l'on a représenté sur la figure 4 une telle seringue équipée du dispositif selon l'invention, avec le piston (7) au repos.

Avantageusement le fourreau présente à chaque extrémité des crochets (8) pour agripper la collerette (6) de la seringue de façon à solidariser le fourreau et la seringue, notamment pour un usage unique.

Il est en effet prévu de préférence que la seringue équipée soit à usage unique, l'utilisateur décidant a priori d'injecter un mandrin gazeux ou un mandrin liquide et plaçant définitivement le fourreau dans la position qui convient pour l'usage envisagé.

Le dispositif est susceptible de nombreuses variantes, dans lesquelles, par exemple :
- les bras ne sont élastiques que sur une partie de leur longueur ;
- l'embase présente plusieurs endroits de fixation possible répartis dans la longueur de l'embase pour les liens à extension élastique.

L'invention n'est pas limitée à la réalisation qui a été décrite.

## Revendications

1. Dispositif pour équiper une seringue à usage médical en sorte que le piston (7) de la seringue soit soumis, lorsqu'il a été tiré pour le remplissage de la seringue, à une pression positive créée par une force de rappel antagoniste due à l'extension d'un lien élastique provoquée par le tirage du piston, qui comprend un poussoir (2) à appliquer sur la tête du piston et une embase (1) à enfiler sur le corps tubulaire (5) de la seringue jusqu'à venir en butée contre une extension latérale (6) de ce corps, ledit poussoir (2) et ladite embase (1) étant reliés par des liens à extension élastiques (3) disposés pour maintenir le poussoir appliqué contre la tête (8) du piston (7) et à se tendre élastiquement lorsque le piston est tiré dans le sens du remplissage de la seringue, **caractérisé en ce que** les liens à extension élastique (3) constituent deux bras parallèles qui relient le poussoir (2) à l'embase (1), **en ce que** l'embase (1) constitue un fourreau, **en ce que** les bras sont montés à pivotement sur le fourreau, et **en ce que** les pivots (4) de fixation des bras (3) sont plus proches d'une extrémité du fourreau que de l'autre extrémité du fourreau et le fourreau peut être enfilé sur le corps tubulaire (5) de la seringue, au choix, par l'une ou par l'autre de ces extrémités, en sorte que l'embase peut être enfilée sur le corps tubulaire par son extrémité (1a) proche des pivots ou par son extrémité (1b) éloignée des pivots

2. Dispositif selon la revendication 1 dont l'embase (1), le poussoir (2) et les liens à extension élastique (3) forment un corps unitaire.

3. Dispositif selon l'une des revendications 1 à 2 dans lequel l'embase présente des crochets (8) pour sa fixation au corps de la seringue.

4. Une seringue équipée d'un dispositif selon l'une des revendications 1 à 3.

## Claims

1. A device for fitting out a syringe intended for medical use so that the piston (7) of the syringe is submitted when it has been drawn for filling the syringe, to a positive pressure generated by an antagonistic restoring force due to the extension of an elastic tie caused by the drawing of the piston, which comprises a pusher (2) to be applied onto the head of the piston and a base (1) to be slipped onto the tubular body (5) of the syringe until it abuts against a side extension (6) of this body, said pusher (2) and said base (1) being connected by elastic extension ties (3) positioned for maintaining the pusher applied against the head (8) of the piston (7) and to be elastically tensioned when the piston is drawn in the direction for filling the syringe, **characterized in that** the elastic extension ties (3) form two parallel arms which connect the pusher (2) to the base (1), **in that** the base (1) forms a sheath, **in that** the arms are pivotably mounted on the sheath, and **in that** the pivots (4) for attaching the arms (3) are closer to one end of the sheath than to the other end of the sheath and the sheath may be slipped onto the tubular body (5) of the syringe, optionally, by either one of these ends, so that the base may be slipped onto the tubular body by its end (la) close to the pivots or by its end (1b) away from the pivots.

2. The device according to claim 1, the base (1), the pusher (2) and the elastic extension ties (3) of which form a unitary body.

3. The device according to any of claims 1 to 2, wherein the base has hooks (8) for attaching it to the body of the syringe.

4. A syringe fitted out with a device according to any of claims 1 to 3.

## Patentansprüche

1. Vorrichtung zur Ausstattung einer Spritze für medizinische Zwecke, so dass der Kolben (7) der Spritze, wenn er zum Befüllen der Spritze gezogen wurde, unter einen positiven Druck gesetzt wird, der von einer antagonistischen Rückholkraft erzeugt wird, die auf die Ausdehnung einer elastischen Verbindung, die vom Ziehen des Kolbens hervorgerufen wird, zurückzuführen ist, die einen Drücker (2) umfasst, der auf den Kopf des Kolbens anzulegen ist und einen Sockel (1), der auf den röhrenförmigen Körper (5) der Spritze aufzustecken ist, bis er gegen eine seitliche Erweiterung (6) dieses Körpers anschlägt, wobei der Drücker (2) und der Sockel (1) durch elastisch ausdehnbare Verbindungen (3) verbunden sind, die angeordnet sind, um den Drücker gegen den Kopf (8) des Kolbens (7) angelegt zu halten und sich elastisch zu spannen, wenn der Kolben in die Richtung des Befüllens der Spritze gezogen wird, **dadurch gekennzeichnet, dass** die elastisch ausdehnbaren Verbindungen (3) zwei parallele Arme bilden, die den Drücker (2) mit dem Sockel (1) verbinden, **dadurch**, dass der Sockel (1) eine Stulpmanschette bildet, **dadurch**, dass die Arme drehbar auf der Stulpmanschette befestigt sind und **dadurch**, dass die Zapfen (4) zur Befestigung der Arme (3) näher an einem Ende der Stulpmanschette sind als am anderen Ende der Stulpmanschette und dass die Stulpmanschette auf den röhrenförmigen Körper (5) der Spritze wahlweise über das eine oder das andere dieser Enden aufsteckbar ist, so dass der Sockel auf den röhrenförmigen Körper über sein Ende (la) in der Nähe der Zapfen oder über sein Ende (1b) in Entfernung der Zapfen aufsteckbar ist.

2. Vorrichtung nach Anspruch 1, wobei der Sockel (1), der Drücker (2) und die elastisch ausdehnbaren Verbindungen (3) einen einheitlichen Körper bilden.

3. Vorrichtung nach einem der Ansprüche 1 bis 2, wobei der Sockel für seine Befestigung am Körper der Spritze Haken (8) aufweist.

4. Eine Spritze, die mit einer Vorrichtung nach einem der Ansprüche 1 bis 3 ausgestattet ist.
